(19)

Europäisches
Patentamt
European
Patent Office
Office européen
des brevets

(11) **EP 2 346 493 B1**

(12) **EUROPÄISCHE PATENTSCHRIFT**

(45) Veröffentlichungstag und Bekanntmachung des
Hinweises auf die Patenterteilung:
**06.01.2016 Patentblatt 2016/01**

(21) Anmeldenummer: **09778566.1**

(22) Anmeldetag: **16.09.2009**

(51) Int Cl.:
***A61K 9/20*** *(2006.01)*

(86) Internationale Anmeldenummer:
**PCT/EP2009/006707**

(87) Internationale Veröffentlichungsnummer:
**WO 2010/034425 (01.04.2010 Gazette 2010/13)**

(54) **MULTIPARTIKULÄRE TABLETTEN UND VERFAHREN ZU DEREN HERSTELLUNG**

MULTIPARTICULATE TABLET AND METHOD FOR THE PRODUCTION THEREOF

COMPRIMÉS MULTIPARTICULAIRES ET LEUR PROCÉDÉ DE FABRICATION

(84) Benannte Vertragsstaaten:
**AT BE BG CH CY CZ DE DK EE ES FI FR GB GR
HR HU IE IS IT LI LT LU LV MC MK MT NL NO PL
PT RO SE SI SK SM TR**

(30) Priorität: **24.09.2008 DE 102008048729**

(43) Veröffentlichungstag der Anmeldung:
**27.07.2011 Patentblatt 2011/30**

(73) Patentinhaber: **ADD Technologies Ltd.
4133 Pratteln (CH)**

(72) Erfinder:
• **GERBER, Frédéric
F-68730 Blotzheim (FR)**

• **SCHLUETERMANN, Burkhard
79280 Au (DE)**

(74) Vertreter: **Henkel, Breuer & Partner
Patentanwälte
Erika-Mann Strasse 23
80636 München (DE)**

(56) Entgegenhaltungen:
EP-A- 1 627 633     WO-A-02/19991
WO-A-03/080032     WO-A-2006/061069
WO-A-2007/016948     DE-A1- 10 104 880

EP 2 346 493 B1

**Beschreibung**

Die Erfindung betrifft multipartikuläre Tabletten und Verfahren zu deren Herstellung. Die Erfindung betrifft ferner ein Granulationsverfahren zum Herstellen sphärischer Partikel zur Verwendung in den multipartikulären Tabletten und anderen multipartikulären Darreichungsformen.

Bei multipartikulären (Multiple-Unit-) Arzneiformen wird die Dosis eines oder mehrerer Wirkstoffe auf viele (in der Regel mehr als Tausend Untereinheiten) verteilt, welche von den jeweiligen einen oder mehrere Wirkstoffe enthaltenden Partikeln gebildet werden. Bei diesen Untereinheiten bzw. Partikeln kann es sich etwa um Pellets bzw. sphärische Partikel handeln. Diese weisen typischerweise entweder einen Schicht-artigen Aufbau oder einen Matrixaufbau auf.

Sowohl Schicht- als auch Matrixpellets können jeweils abschließend mit einem oder mehreren Filmen beschichtet werden. Die Beschichtung mit einem Film kann, falls erforderlich, aus unterschiedlichen Zwecken verfolgt werden, so zum Beispiel zur Verbesserung der Verarbeitbarkeit, etwa durch Verbesserung des Fließverhaltens oder Verminderung der Hygroskopie, zur Gewährleistung und Verbesserung der chemischen und/oder mechanischen Stabilität des Arzneistoffes, zur Maskierung eines schlechten Geschmacks oder eines unangenehmen Geruchs oder zur Veränderung der Lösungs- bzw. Freisetzungsgeschwindigkeit des oder der inkorporierten Wirkstoffe.

Die multipartikulären Darreichungsformen sind besonders bei modifiziert freisetzenden Systemen von Interesse, da sie vor allem aus biopharmazeutischen Gründen Vorteile gegenüber den sogenannten Single-Unit Arzneiformen aufweisen. Single-units sind monolithische Arzneiformen, d.h. modifiziert freisetzende Einzelarzneiformen, die den Magen-Darm-Trakt unzerfallen passieren, durch Abbau, Erosion, immer kleiner werden oder erst im Darm die Arzneistoffe freisetzen (R. Voigt, Pharmazeutische Technologie, 8. Auflage, Berlin, 1995). Im Gegensatz dazu zerfallen die multipartikulären Systeme in ihre Untereinheiten, welche jeweils ein gewünschtes gesteuertes Freisetzungsverhalten aufweisen können. Eine Beschädigung der Tablette oder eine Fehlfunktion im Hinblick auf die Freisetzung wirkt sich bei multipartikulären Arzneiformen daher üblicherweise, wenn überhaupt, deutlich weniger negativ aus als bei etwa einer Film-ummantelten oder auch Matrixform einer Single-unit-Präparation.

Unter den pharmazeutisch eingesetzten Darreichungsformen besitzen die oralen Zubereitungen nach wie vor die größte Bedeutung. Diese stellen für den Patienten die angenehmsten und gebräuchlichsten pharmazeutischen Zubereitungen dar. Multipartikuläre Arzneiformen können zur Applikation in Kapseln, z.B. Hartgelatinekapseln, oder in Sachets abgefüllt werden, oder, was aus galenischen Gesichtspunkten am anspruchsvollsten ist, zu Tabletten verpresst vorliegen. Oft wird insbesondere bei Tabletten zur Erleichterung der Schluckbarkeit, oder um den Aspekt einer solchen Darreichungsform zu verbessern, ein zusätzlicher Film aufgetragen. Die häufigste Arzneiform unter diesen festen Arzneimitteln ist die Tablette, die gegenüber einer Hartgelatinekapsel nicht nur eine tendenziell verbesserte Schluckbarkeit aufweist, sondern auch ökonomischer hergestellt werden kann.

Die Komprimierung von multipartikulären Systemen, insbesondere von überzogenen multipartikulären Systemen ist allerdings nicht einfach, da viele Faktoren die Qualität der Arzneiform auch wechselseitig beeinflussen können. Beispielsweise sind dünne Filme im allgemeinen schneller freisetzend und werden aber auch bei einer nachfolgenden Tablettierung zu Tabletten leichter durch die aufgewendete Presskraft zerstört.

Wichtige Faktoren, die bei der Herstellung einer qualitativ hochwertigen multipartikulären Darreichungsform eine Rolle spielen, sind neben a) der Auswahl geeigneter pharmazeutischer Hilfsstoffe, und b) der Wahl geeigneter, gut kontrollierter Herstellungsprozesse, auch c) der Aufbau einer Tablette.

Im Prinzip entspricht die Komposition einer multipartikulären Tablette im Wesentlichen dem nachfolgend geschilderten Aufbau einer klassischen Tablettenformulierung zur sofortigen Freisetzung:

Diese besteht a) aus einer Innenphase und b) einer Außenphase.

Die Innenphase, auch innere Phase genannt, einer Tablette enthält den Wirkstoff, meistens in granulierter, d.h. aggregierter Form. Als Hilfsstoffe enthält die innere Phase typischerweise als Hauptbestandteile Füllmittel, Bindemittel und, falls erforderlich, Sprengmittel und/oder weitere Hilfsstoffe.

Die Außenphase einer Tablette, oft als äussere Phase bezeichnet, enthält typischerweise in feinpulveriger und nicht-aggregierter Form beispielsweise Sprengmittel, Schmiermittel und Fliessregulierungsmittel, eventuell aber auch noch geringe Mengen Füll- und Bindemittel. Zusätzlich können Geschmacksstoffe und weitere Zusatzstoffe enthalten sein. Der Begriff Außenphase ist funktional, nicht räumlich zu verstehen. Eine gute Übersicht gängiger Hilfsstoffe findet sich bei H.P. Fiedler, Lexikon der Hilfsstoffe für Pharmazie, Kosmetik und angrenzende Gebiete, Editio Cantor Verlag Aulendorf.

Bei einer klassischen Tablettenformulierung ist, einer Faustregel zur Folge, das Verhältnis zwischen Innenphase und Außenphase, im Hinblick auf optimale Fliesseigenschaften und Packungsdichten etwa 8:2 und 9:1 (K.H. Bauer, K.-H. Frömming, C. Führer, Pharmazeutische Technologies, Thieme, 1986).

Bei einer multipartikulären, festen Arzneiform ist das Phasenverhältnis in der Regel anders. Hier ist der Anteil der Außenphase deutlich erhöht. Bei akzeptablen, multipartikulären, hochdosierten Zubereitungen sollte das Phasenverhältnis von Innenzu Außenphase im Bereich von 3:7, mindestens jedoch 4:6., wenn nicht höher sein (M. Braun, Dissertation, 2003, Rheinische-Friedrich-Wilhelms-Universität Bonn; K.G. Wagner, Dissertation, 1999, Eberhard-Karls-Universität Tübingen).

Es ist dabei von besonderer Bedeutung, dass durch die anteilig erhöhte Außenphase die Innenphase der Tablettenformulierung, die in der Regel die wirkstoffhaltigen Partikel enthält, vor der Krafteinwirkung beim Tablettieren geschützt wird. Durch die Krafteinwirkung bzw. die sich daraus ergebende mechanische Belastung kann eine Veränderung der Produkteigenschaften resultieren. Dies gilt insbesondere bei den hier beschriebenen multipartikulären Partikelsystemen, die dadurch gekennzeichnet sind, dass die Produkteigenschaften dieser Darreichungsform auf der Stufe der jeweiligen Untereinheiten definiert werden und diese Untereinheiten durch die Tablettierung nach Möglichkeit nicht nachteilig beeinflusst werden sollen.

Um diese zu erreichen, ist das Phasenverhältnis von Innen- zur Außenphase bei solchen Darreichungsformen in der Regel deshalb zugunsten der Außenphase erhöht, um u.a. bei ausreichender Gewichts- und Gehaltseinheitlichkeit der Darreichungsform noch Pellets vor Schäden durch die mechanische Belastung bei der Tablettierung zu schützen. Die Tablettierung kann eine Veränderung der Produkteigenschaften bewirken.

Insbesondere Darreichungsformen, in welchen die Partikel jeweils mit einem Film bzw. Funktionslack beschichtet sind, enthalten im Gegensatz zu den zuvor genannten klassischen Tabletten einen deutlich erhöhten Anteil an Außenphase, um Beschädigungen am Film eines überzogenen Pellets infolge der Krafteinwirkung bei der Tablettierung zu reduzieren, um die Produkteigenschaften der inkorporierten Pellets bzw. überzogenen Arzneistoffe nicht zu verändern, eine ausreichende mechanische Stabilität der Tablette, ein akzeptables Freisetzungsverhalten, aber auch eine ausreichende Gewichts- und Gehaltseinheitlichkeit zu gewährleisten. Die Anforderungen an die Gehaltseinheitlichkeit einer Darreichungsform ist bei den multipartikulären Darreichungsformen besonders kritisch. Die Prüfung auf die Gleichförmigkeit des Gehaltes und die Anforderungen an Darreichungsformen sind in den aktuellen Arzneibüchern hinreichend dokumentiert.

Die Verarbeitung von überzogenen Wirkstoffen ist ähnlich kritisch wie die der zuvor beschriebenen Pellets. Deshalb sind im Sinne dieser Spezifikation überzogene Wirkstoffe, die nachfolgend zu einer Tablette verarbeitet werden, auch Gegenstand der hier weiter ausgeführten Erfindung.

Der Anteil an Außenphase kann also in diesen Fällen bis zu 70 % oder sogar mehr betragen, wobei der am häufigsten eingesetzte Hilfsstoff faserige, mikrokristalline Zellulose ist, in die Pellets oder überzogene Wirkstoffe besonders stabil eingebettet werden können.

Infolge der Krafteinwirkung bei der Tablettierung können also beispielsweise die Freisetzungseigenschaften der Pellets in einer Tablette nachteilig verändert werden, so dass sich die Freisetzung des Arzneistoffs z.B. beschleunigt, eine Magensaftresistenz verloren geht, oder eine bestehende Geschmacksmaskierung gänzlich aufgehoben wird. Diese Nachteile sind umso kritischer, wenn die zu verabreichenden Arzneistoffdosen hoch sind, wenn Profile mit unterschiedlicher Freisetzungsrate oder Art der Freisetzung kombiniert werden sollen und/oder wenn mehrere Arzneistoffe miteinander kombiniert zu einer Darreichungsform verarbeitet werden sollen. Letztere Präparate sind als Kombinationspräparate - oder auch Fixkombinationen genannt - Arzneimittel mit mehreren Wirkstoffen.

Eine Kombination von mehreren Wirkstoffen kann insbesondere dann sinnvoll sein, wenn nachgewiesen ist, dass jeder einzelne Wirkstoff in Bezug auf das beanspruchte Anwendungsgebiet therapeutisch wirksam ist und die Dosierung jedes einzelnen Inhaltsstoffs im Hinblick auf die Höchstdosierung, die Anwendungshäufigkeit und -dauer so bemessen ist, dass eine nennenswerte Patientenzahl einer solchen fixen Kombination bedarf und sie wirksam und unbedenklich im Sinne eines Nutzen- und Risikoverhältnisses ist, und die zugeführten Wirkstoffe die Wirksamkeit und/oder Unbedenklichkeit der Wirkstoffe bzw. des Hauptwirkstoffs erhöhen oder die Möglichkeit des Missbrauchs des Hauptinhaltsstoffes verringern oder die fixe Kombination von Wirkstoffen einen grösseren therapeutischen Effekt hervorruft oder grössere Unbedenklichkeit bietet als jeder einzelne Wirkstoff für sich. Diese Kriterien, die als Croutsche Kriterien bekannt sind, sind auch im Arzneimittelgesetz berücksichtigt.

So gibt es viele Beispiele für sehr sinnvolle Kombinationspräparate, die zwei oder mehrere Arzneistoffe enthalten, die sich u.a. in ihrer Wirkung gegenseitig unterstützen. Insbesondere älteren Menschen fällt es schwer, sich an die Einnahme von verschiedenen Medikamenten zu gewöhnen. Die Einnahme eines Kombinationspräparates erleichtert und gewährleistet in vielen Fällen, u.a. auch in der Pädiatrie, die Therapie und verbessert somit die Compliance, und trägt zur Arzneimittelsicherheit bei.

Kombinationspräparate werden sehr häufig eingesetzt u.a. in der Behandlung von Bluthochdruck, zur Behandlung der Parkinsonschen Krankheit, zur Behandlung von Erkrankungen des zentralen Nervensystems, zur Infektabwehr mit Antibiotika oder antiviral wirksamen Substanzen, zur oralen Empfängnisverhütung, zur Behandlung von Magenerkrankungen und in der Schmerztherapie.

Masse und Dimensionen von Kombinationspräparaten oder generell auch von multipartikulären Darreichungsformen unterscheiden sich, wenn man von sehr niedrig dosierten Zubereitungen, wie z.B. den Hormonpräparaten zur Empfängnisverhütung einmal absieht, üblicherweise von den entsprechen Darreichungsformen des einzelnen Arzneistoffs und dessen schnell freisetzenden Darreichungsformen.

Dies gilt insbesondere dann, wenn der Arzneistoff oder mehrere Arzneistoffe eines Arzneimittelkombinationsproduktes höher dosiert ist (sind) oder die Auflösungsgeschwindigkeit eines oder mehrerer Wirkstoffe modifiziert werden soll. Dies gilt aber auch für Produkte mit geschmacksmaskierten Wirkstoffen oder Pellets, die zur Geschmacksmaskierung Überzüge aufweisen und anschliessend zu Tabletten, insbesondere zu oral zerfallenden Tabletten verarbeitet werden sollen.

Bisher war es unmöglich oder sehr schwierig, neben überzogenen Pellets oder überzogenen Wirkstoffen gleichzeitig weitere Arzneistoffe in höher dosierter Form zu Kombinationsprodukten zu verarbeiten, da hinsichtlich Masse und Dimensionen nicht mehr einfach zu applizierende, das heisst schluckbare Tabletten resultierten oder sich die Produkteigenschaften der einzelnen Pelletsorten auf nicht akzeptable Weise veränderten.

Es ist daher eine Aufgabe der vorliegenden Erfindung, eine multipartikuläre Tablette mit einer verringerten Menge an Außenphase bereitzustellen. Es ist eine weitere Aufgabe der vorliegenden Erfindung, eine multipartikuläre Tablette mit einer Kombination von Wirkstoffen bereitzustellen. Eine weitere Aufgabe der vorliegenden Erfindung ist es, eine multipartikuläre Tablette mit einem vergleichsweise hohen Wirkstoffgehalt bereitzustellen.

Darüber hinaus bestehen Schwierigkeiten bei der Herstellung von Multiple-Unit Arzneiformen, insbesondere wenn z.B. Fixkombinationen als multipartikuläre Darreichungsformen formuliert werden sollen.

Bisher bekannte Granulationsprozesse sind häufig nicht zufriedenstellend, da ein hoch dosierter Wirkstoff zu seiner Verarbeitung mit geeigneten Hilfsstoffen zu sehr verdünnt werden muss oder unzureichende Prozesstechnologien zur Verfügung stehen, um hinsichtlich Verarbeitbarkeit, Grösse, Masse, Zerfall, Härte und Freisetzung akzeptable Eigenschaften von Zwischen- und Endprodukten zu erhalten.

Bekannte Granulationsprozesse haben ausserdem den Nachteil, dass Wirkstoffe, die bei Einsatz von wenig zusätzlichen Hilfsstoffen, wie z.B. Füll-, Binde-, Spreng-, Fliessregulierungs und Schmiermittel ( z.B. < 60 Gew.%) sehr empfindlich auf feuchte und trockene Aggregationsprozesse reagieren, den Wirkstoff aus den entsprechenden Granulaten bzw. finalen Darreichungsformen nur unzureichend und/oder wenig steuerbar freisetzen. Durch solche Granulationsverfahren entstehen häufig hochverdichtete Agglomerate, die den Wirkstoff nachfolgend nur ungenügend freisetzen. Beispiele dafür sind beispielsweise Ascorbinsäure und Oxcarbazepin.

Der typische Granulationsprozess für einen Arzneistoff ist vielschichtig. Nach Mischen, Aggregieren durch Befeuchten, Kneten, Erwärmen oder Druck, und der einer Feuchtgranulation nachfolgenden Trocknung, müssen die erhaltenen Granulate entsprechend ihren Erfordernissen wieder zerkleinert und klassiert werden.

Es war daher eine weitere Aufgabe der vorliegenden Erfindung, ein gut steuerbares Granulationsverfahren und entsprechende Formulierungen auch für hoch dosierte Wirkstoffe in Kombination mit Pelletprodukten bzw. überzogenen Wirkstoffen (I) mit hohem Wirkstoffanteil bereitzustellen, um gleichzeitig gute Verarbeitungs-, Zerfalls- und Auflösungseigenschaften aller arzneilich wirksamen Bestandteile einer Darreichungsform zu ermöglichen, die bei der Herstellung die Eigenschaften der inkorporierten Pellets (I), insbesondere die der Dissolution, im Wesentlichen unverändert lassen.

Die vorstehend genannten Aufgaben werden durch erfindungsgemäßen Tabletten und Verfahren gelöst. Vorteilhafte Ausführungsformen sind nachfolgend beschrieben und Gegenstand der abhängigen Ansprüche.

Die Erfindung betrifft nun gemäß einem ersten Aspekt eine Tablette, enthaltend eine Innenphase, umfassend

a) eine erste Sorte von Pellets (I), welche einen ersten Wirkstoff enthalten und welche eine Beschichtung aufweisen und/oder den Wirkstoff modifiziert freisetzen, oder

b) eine erste Sorte überzogenen ersten Wirkstoffs (I), im Gemisch mit

c) sphärischen Partikeln (II),

und eine Außenphase mit einem oder mehreren vor der Tablettierung nicht granuliert vorliegenden Hilfsstoffen, ausgewählt aus Sprengmitteln, Schmiermitteln, Fliessregulierungsmitteln, Füllmitteln und Bindemitteln,

wobei das Gewicht der Außenphase nicht mehr als 25 Prozent des Gesamtgewichts der Tablette, beispielsweise nicht mehr als 20%, 15%, 10% oder 5% ausmacht.

Das Gewicht der Außenphase kann beispielsweise bis zu 25%, 22,5%, 20%, 17,5%, 15% 12,5%, 10%, 7,5%, 5%, 4%, 3%, 2% oder 1% des Gesamtgewichts der Tablette ausmachen. Das Gewicht der Außenphase kann beispielsweise mindestens 0,5%, 1%, 2%, 3% oder 5% des Gesamtgewichts der Tablette ausmachen.

Als vor Tablettierung nicht granuliert vorliegende Hilfsstoffe für die Außenphase können geeignete übliche Hilfsstoffe verwendet werden (s. H.P. Fiedler, Lexikon der Hilfsstoffe für Pharmazie, Kosmetik und angrenzende Gebiete, Editio Cantor Verlag Aulendorf). Zusätzlich können Geschmacksstoffe, Farbstoffe, Solubilisatoren und weitere Zusatzstoffe in der Außenphase enthalten sein.

Das Gewicht der ersten Sorte Pellets (I) oder der ersten Sorte des überzogenen ersten Wirkstoffs (I) kann beispielsweise 35 bis 55% des Gesamtgewichts der Tablette ausmachen, beispielsweise von 37,5 bis 52,5% oder von 40 bis 50%. Werden mehrere Sorten Pellets (I) oder überzogener Wirkstoffe (I) verwendet, so gilt dies entsprechend für deren Gesamtgewicht.

Das Gewicht der sphärischen Partikel (II) in den Ausführungsformen, die außerdem mindestens eine erste Sorte Pellets (I) und/oder überzogenen Wirkstoff (I) enthalten, kann beispielsweise im Bereich von 30% bis 70%, bevorzugt im Bereich von 35 bis 65%, oder 40 bis 60% des Gesamtgewichts der Tablette liegen.

Damit werden multipartikuläre Tabletten bereitgestellt, deren Innenphase ein erstes Pelletprodukt oder überzogenen Wirkstoff (I) enthält und deren Außenphase gegenüber einer herkömmlichen multipartikulären Darreichungsformen dadurch deutlich reduziert werden kann, dass zu dem ersten Pelletprodukt (I) bzw. dem überzogenen Wirkstoff sphärische Partikel (II) zugemischt werden.

Generell muss die Außenphase einer Multiple-Unit Tablette kinetische Energie, die durch die Tablettierung ins Produkt gelangt, aufnehmen. Sie trägt deshalb wesentlich dazu bei, dass mechanisch ausreichend stabile Tabletten entstehen. Für die Güte der Außenphase einer multipartikulären Tablette, die unter möglichst schonenden Bedingungen hergestellt werden soll, ist ein ausgewogenes Verhältnis von elastischer und plastischer Verformbarkeit erforderlich.

Die sphärischen Partikel (II), welche allen Ausführungsformen dieser Erfindung gemeinsam sind, weisen vorteilhafte Eigenschaften auf, insbesondere ein ausgewogenes Verhältnis elastischer und plastischer Eigenschaften, welche überraschenderweise eine Einsparung an Außenphase ermöglichen. Neben der Komprimiereigenschaften dieser Partikel sind deren technologische Kennzahlen, wie Form, Dichte und Porosiät, zur Substitution der Außenphase für multipartikuläre Tabletten ebenfalls von Bedeutung.

Ein bevorzugtes Herstellungsverfahren und die vorteilhaften Eigenschaften der sphärischen Aggregate (II) werden nachfolgend noch näher beschrieben.

Die sphärischen Partikel (II), die den Pellets (I) oder überzogenen Wirkstoffen (I) zugesetzt werden, können allein aus (pharmazeutischen) Hilfsstoffen bestehen. Beispiele für Hilfsstoffe, die vorteilhaft eingesetzt werden können, werden nachstehend im Zusammenhang mit einem bevorzugten Granulationsverfahren und im Rahmen der Ausführungsbeispiele beschrieben.

Die Pellets (I) und die sphärischen Partikel (II) unterscheiden sich hinsichtlich mindestens eines Parameters voneinander, etwa hinsichtlich ihrer Zusammensetzung, ihres Aufbaus und/oder hinsichtlich eines physikalischen Parameter, der z.B. durch die Herstellung beeinflusst sein kann, wie z.B. Dichte, Porosität etc..

Bei den Pellets (I) handelt es sich vorzugsweise um sphärische Pellets. Die Pellets (I) können nach dem gleichen Verfahren hergestellt sein wie die sphärischen Partikel (II), dann enthält die Zusammensetzung zwei Sorten sphärischer Produkte bzw. Partikel (II). Sie können aber auch nach einem anderen Verfahren hergestellt sein.

Die erste Sorte Pellets ist in besonders bevorzugten Ausführungsformen so beschaffen, dass sie den in ihnen enthaltenen Wirkstoff modifiziert freisetzen.

Die erfindungsgemäß verwendeten Pellets können eine Beschichtung aufweisen, beispielsweise einen Filmüberzug. Die Beschichtung kann die für solche Beschichtungen üblichen Funktionen haben. Die Beschichtung kann beispielsweise eine Beschichtung zur Geschmacksmaskierung sein, eine Beschichtung zur Geruchsmaskierung, eine Beschichtung zur Stabilisierung des Wirkstoffs, eine Beschichtung zur Verbesserung der Verarbeitbarkeit, eine Beschichtung zur Verbesserung des Fließverhaltens, eine Beschichtung zur Verminderung der Hygroskopie, eine Beschichtung zur Gewährleistung und Verbesserung der chemischen und/oder mechanischen Stabilität des Arzneistoffes.

Besonders bevorzugt ist mindestens die erste Sorte Pellets (I) mit einer Beschichtung versehen, welche die Freisetzung des Wirkstoffs aus den Pellets (I) modifiziert. Modifizierung der Freisetzung umfasst beispielsweise gleichmäßig hinhaltende Wirkstofffreigabe, verlängerte Wirkstofffreigabe, verzögerte Wirkstofffreigabe, gestaffelte Wirkstofffreigabe und Kombinationen davon (controlled release, extended release, prolonged release, repeated release, delayed release).

Beispiele für Materialien, die für derartige Beschichtungen geeignet sind, umfassen Cellulosederivate, wie z.B. Ethylcellulose, Methylcellulose, Hydroxypropylmethylcellulose, Celluloseacetatphthalat, Polymere aus Methacrylsäure und Methacrylsäureestern, wie z.B.Eudragit, oder Polyvinylderivate,

Alternativ kann eine Modifizierung der Freisetzung aus den Pellets statt durch eine Beschichtung auch durch andere im Fachbereich übliche Maßnahmen erreicht werden, etwa indem die Pellets als Matrix aufgebaut sind, welche eine Modifizierung der Freisetzung erlaubt.

Besonders vorteilhaft sind allgemein diejenigen Ausführungsformen von Tabletten der vorliegenden Erfindung, in denen die Pellets (I) mit einem Film beschichtet sind, d.h. die Beschichtung als Film vorliegt.

Entsprechend den obigen Ausführungen zu den Beschichtungen kann es sich beispielsweise um einen geschmacksmaskierenden Film oder einen geruchsmaskierenden Film handeln. Noch vorteilhafter sind diejenigen Ausführungsformen, in welchen die Pellets (I) mit einem Film überzogen sind, welcher die Freisetzung des Wirkstoffs (bzw. der Wirkstoffe) aus dem Pellet (I) modifiziert. Dabei kann es sich um die üblichen im Fachbereich bekannten Filme bzw. Funktionslacke handeln. Mögliche Funktionen solcher Filme bzw. Funktionslacke sind beispielsweise die Retardierung der Wirkstofffreisetzung und die Herstellung von Magensaftresistenz. Der Filmüberzug kann beispielsweise eine enterische Beschichtung sein.

Analoges gilt für den Überzug der überzogenen Wirkstoffe, insbesondere kann der Überzug die gleichen Funktionen wie die Beschichtung der Pellets ausüben, beispielsweise die Freisetzung modifizieren. Es wird daher auf die oben genannten Beispiele für Beschichtungen verwiesen.

Beschichtungen, Überzüge und Filme sind typischerweise Wirkstoff-frei. Es sind jedoch auch Ausführungsformen denkbar, in welchen Wirkstoff in der Beschichtung enthalten ist.

In beispielhaften Ausführungsformen können eine erste Sorte Pellets (I) mit einem ersten Filmüberzug bzw. einer ersten Beschichtung und eine zweite Sorte Pellets (I) mit einem zweiten Filmüberzug bzw. einer zweiten Beschichtung eingesetzt werden, wobei die beiden Sorten Pellets (I) den gleichen Wirkstoff enthalten, sich aber bezüglich ihres Freisetzungsverhaltens voneinander unterscheiden. Eine Pelletsorte kann beispielsweise mit einem die Freisetzung modifizierenden, insbesondere verlangsamenden bzw. verzögernden Film beschichtet sein, während die andere Pelletsorte den Wirkstoff sofort freisetzt. Damit kann eine vorteilhafte Dauer und Kontinuität der Wirkstofffreisetzung erreicht werden.

Es sind auch Ausführungsformen denkbar, in denen die erste Sorte Pellets mit dem ersten Wirkstoff in Kombination mit einer ersten Sorte überzogenen ersten Wirkstoffs vorliegt. In solchen Ausführungsformen können z.B. die erste Sorte Pellets und die erste Sorte überzogenen Wirkstoffes ein unterschiedliches Freisetzungsverhalten (Dissolutionsprofil) dieses gleichen Wirkstoffs aufweisen. Beispielsweise kann die erste Sorte Pellets, wie bereits erwähnt, mit einem die Freisetzung beeinflussenden, insbesondere verlangsamenden Film überzogen sein, während der überzogene Wirkstoff den Wirkstoff sofort freisetzt. Alternativ kann der Überzug des Wirkstoffs die Freisetzung verlangsamen.

Es sind ferner Ausführungsformen denkbar, in welchen neben der erste Sorte Pellets (I) eine zweite Sorte Pellets mit einem zweiten Wirkstoff vorliegen. Damit lassen sich Kombinationspräparate verwirklichen, in welchen durch den verminderten Bedarf an Außenphase die Tablettendimensionen verringert werden und somit die Schluckbarkeit verbessert werden kann. Auch hier können verschiedene Beschichtungen bzw. Filmüberzüge verwendet werden. Durch geeignete Auswahl von Filmen bzw. Pelletaufbau bzw. -Zusammensetzung lassen sich auch ansonsten miteinander unverträgliche Wirkstoffe als Kombinationspräparat in einer Tablette verwirklichen.

In allen diesen Varianten können die sphärischen Partikel Wirkstoff-frei sein. Alternativ können die sphärischen Partikel (I) auch einen oder mehrere Wirkstoffe enthalten. Hierdurch können besonders vorteilhafte Kombinationspräparate erhalten werden. Die sphärischen Aggregate (II) können unbeschichtet sein oder ebenfalls mit einer Beschichtung versehen sein, die obigen Ausführungen zu möglichen Beschichtungen der Pellets (I) gelten hier analog.

Beispielsweise sind Ausführungsformen denkbar, in welchen die erste Sorte Pellets (I) und die sphärischen Partikel (II) den gleichen Wirkstoff enthalten, jedoch unterschiedliche Freisetzungsprofile aufweisen. Beispielsweise kann die erste Pelletsorte (I) mit einem die Freisetzung modifizierenden, insbesondere verlangsamenden und/oder verzögernden Film beschichtet sein, während die sphärischen Partikel (II) den Wirkstoff sofort freisetzen. Damit kann eine vorteilhafte Dauer und Kontinuität der Wirkstofffreisetzung erreicht werden.

Die sphärischen Partikel können auch einen zweiten Wirkstoff oder mehrere Wirkstoffe enthalten, welche(r) beispielsweise von dem ersten Wirkstoff der Pellets (I) oder der überzogenen Wirkstoff(kristalle) verschieden ist (sind).

Es können so besonders vorteilhaft Kombinationspräparate bereitgestellt werden. Eine bevorzugte Ausführungsform betrifft also eine Tablette, welche enthält:

eine Innenphase, umfassend

a) eine erste Sorte von Pellets (I), welche einen ersten Wirkstoff enthalten und eine Beschichtung aufweisen und/oder den ersten Wirkstoff modifiziert freisetzen, oder
b) eine erste Sorte überzogenen ersten Wirkstoffs (I),

im Gemisch mit

c) sphärischen Partikeln (II), welche einen zweiten Wirkstoff enthalten,

und eine Außenphase mit einem oder mehreren vor der Tablettierung nicht granuliert vorliegenden Hilfsstoffen, ausgewählt aus Sprengmitteln, Schmiermitteln, Fliessregulierungsmitteln, Füllmitteln und Bindemitteln, wobei das Gewicht der Außenphase nicht mehr als 25 Prozent des Gesamtgewichts der Tablette, beispielsweise nicht mehr als 20%, 17,5%, 15%, 12,5%, 10%, 7,5% oder 5% ausmacht.

Analog zu den vorstehend gemachten Ausführungen können auch die sphärischen Partikel mit Film beschichtet bzw. mit Film überzogen sein, und verschiedene Freisetzungsprofile miteinander kombiniert werden, so z.B. Pellets zur sofortigen Freisetzung mit sphärischen Aggregaten, welche mit einem die Freisetzung modifizierenden Film beschichtet sind, oder umgekehrt.

Die sphärischen Partikel (II) werden vorzugweise durch Feuchtgranulation hergestellt, besonders bevorzugt durch Granulation in der Wirbelschicht. Die sphärischen Partikel können insbesondere durch Rotorgranulation in der Wirbelschicht, vorzugsweise durch ein Granulationsverfahren mit gerichteter Gutbewegung und besonders bevorzugt durch Rotorgranulation mit dem Schaufelrotor hergestellt werden.

Ein Beispiel für ein geeignetes Verfahren zur Herstellung dieser runden Granulate bzw. sphärischen Aggregate (II), die einen hohen Wirkstoffanteil aufzeigen können, ist ein Granulationsverfahren, bei dem

a) ein oder mehrere Hilfsstoff(e) und ein oder mehrere Wirkstoff(e), in einem Vertikalgranulator mit einer Flüssigkeit gemischt und vorbefeuchtet werden,

b) die befeuchtete Mischung nachfolgend im Schaufelrotor unter Flüssigkeitszugabe zu einem im wesentlichen sphärischen Aggregat verarbeitet wird,

c) in einer Wirbelschichtanlage bzw. einem anderen geeigneten Maschine getrocknet wird und

d) nachfolgend, nach eventuell erforderlicher Abtrennung von Grobkorn, und nach Zugabe eines oder mehrere Hilfsstoffe und einer ersten Sorte von Pellets (I) oder einer ersten Sorte überzogenen Wirkstoffs (I) zu einem geeigneten pharmazeutischen Zwischen- oder Endprodukt verarbeitet werden kann.

Eine geeignete Vorrichtung und ein geeignetes Verfahren sind grundsätzlich in der internationalen Patentanmeldung WO 2004/052607 A1 beschrieben. Es wurde gefunden, dass sich damit bei Anwendung spezieller Verfahrensbedingungen sphärische Partikel, insbesondere mit hoher Wirkstoffbeladung, herstellen lassen, welche in der vorliegenden Erfindung vorteilhaft zur Anwendung kommen und überraschenderweise eine Einsparung von Außenphase, selbst bei hohen Wirkstoffgehalten, ermöglichen.

Gemäß einer bevorzugten Ausführungsform werden die sphärischen Aggregate wie folg hergestellt:

(a) Einführen des Ausgangsmaterialpulvers, das wahlweise mit einem pharmazeutisch geeigneten flüssigen Verdünnungsmittel benetzt ist, in eine Vorrichtung, die aufweist:

eine Rotorkammer mit einer sich axial erstreckenden zylindrischen Wand,

eine Einrichtung, um Luft durch die Rotorkammer vom Boden aus zu leiten,

eine Sprüheinrichtung für die Zufuhr von Flüssigkeit in die Kammer,

eine oder mehrere Einlassöffnungen zur Einführung des Pulvergemisches,

einen Rotor, der sich um eine vertikale Rotorachse dreht, wobei der Rotor in der Rotorkammer angeordnet ist, eine zentrale horizontale Oberfläche und in mindestens dem äußeren Drittel des Rotors die Form einer konischen Mantelfläche mit einer nach außen und oben gerichteten Neigung zwischen 10° und 80° aufweist, wobei die konische Mantelfläche eine kreisförmige obere Kante aufweist, die in einer Ebene liegt, die senkrecht zu der Rotorachse ist,

eine Mehrzahl von Leitschaufeln jeweils mit einem äußeren Ende, das statisch an der zylindrischen Wand der Rotorkammer oberhalb der Ebene befestigt ist, die von der oberen Kante der konischen Mantelfläche des Rotors gebildet wird, und einem inneren Ende, das sich in die Rotorkammer erstreckt und tangential zu der zylindrischen Wand der Rotorkammer angeordnet ist und im Querschnitt zur Rotorachse im Wesentlichen die Form eines Kreisbogens oder einer Spirale aufweist,

(b) Rotation des Rotors, so dass das Produkt, das durch kinetische Energie für einen ausreichenden Zeitraum im Kreis geführt wird, sich von dem Rotor zu der inneren Oberfläche der Leitschaufeln bewegt, bevor es zurück auf den Rotor fällt, während optional Luft zugeführt und/oder eine pharmazeutisch annehmbare Flüssigkeit in die Rotorkammer gesprüht wird, so dass feste Pellets mit einem gewünschten Durchmesser gebildet werden.

Nach einem analogen Verfahren lassen sich auch entsprechend vorteilhafte sphärische Partikel (II) ohne Wirkstoff herstellen.

Insbesondere Wirkstoffe, die bei Einsatz von wenig zusätzlichen Hilfsstoffen in herkömmlichen Verfahren sehr empfindlich auf feuchte und trockene Aggregationsprozesse reagieren, so dass aus den entsprechenden Granulaten bzw. finalen Darreichungsformen der Wirkstoff nur unzureichend und/oder wenig steuerbar freigesetzt würde, können mit Hilfe des vorstehenden Verfahrens erfolgreich in sphärische Partikel überführt werden, welche es sogar erlauben, den Hilfsstoffanteil in der Tablette um 1/3 oder 1/2 zu reduzieren.

Der oder die in Schritt a) genannten Hilfsstoffe können beispielsweise einen oder mehrere granulationsfördernde(n) Hilfsstoff(e) umfassen.

Entsprechend dem voranstehend erwähnten bevorzugten Herstellungsverfahren bestehen die sphärischen Partikel (II) bevorzugt aus einem oder mehreren geeigneten (pharmazeutischen) Hilfsstoffen, dem bzw. denen ein oder mehrere Wirkstoffe zugesetzt sind. Als (pharmazeutisch) geeignete Hilfsstoffe finden hier insbesondere wasserlösliche, mit Wasser quellbare aber auch wasserunlösliche Hilfsstoffe aus der Gruppe der Füll- und Bindemittel und Zerfall-fördernde Hilfsstoffe (Sprengmittel) Anwendung.

Vorzugsweise ist der Füllstoff eine feine, mikrokristalline Zellulose und/oder ein wasserlösliches Kohlenhydrat wie z.B. Mannitol, Sorbitol oder Xylit, das Bindemittel ein wasserlöslicher, polymerer Hilfsstoff wie Polyvinylpyrrolidon, Hydroxypropylmethylcelullose, Hydroxypropylcellulose, Methylcellulose, Hydroxymethylcellulose, Hydroxyethylcellulose, Hydroxypropylcellulose, Alginate, Pektine, Polyvinylacetate, Xanthane sowie andere Bindemittel, die üblicherweise zur Tablettenherstellung verwendet werden können, sowie Mischungen hiervon.

Der Zerfall-fördernder Hilfsstoff bzw. Sprengmittel ist bevorzugt ausgewählt aus quervernetztem Polyvinylpyrrolidon, quervernetzter Natriumcarboxymethylcellulose und quervernetzter Natriumcarboxymethylstärke, da diese Hilfsstoffe neben ihrer sprengenden Wirkung auch das Aggregieren zu sphärischen Körpern erleichtern, da sie bei der Herstellung als Feuchtigkeitspuffer wirken und somit den Herstellungsprozess stabilisieren.

Als Befeuchtungs- und Granulationsmittel während der Vorbefeuchtung und Aggregation im Schaufelrotor wird vorzugsweise Wasser eingesetzt. In weiteren bevorzugten Varianten werden als Befeuchtungsmittel organische Flüssigkeiten wie Alkohole oder Ketone, wie z.B. Methanol, Ethanol, Isopropanol und Aceton, oder Gemische von Alkoholen und Wasser oder Ketonen und Wasser eingesetzt. Den Befeuchtungsmitteln kann in einer weiteren bevorzugten Variante auch das Bindemittel in gelöster Form zugesetzt werden.

Der kumulative Wirkstoffanteil der daraus hergestellten sphärischen Partikel (II) kann sehr hoch liegen und beispielsweise bis zu 95 % betragen. Es lassen sich also mit dem beschriebenen Granulationsverfahren vorteilhaft sphärische Partikel (II) insbesondere für hoch dosierte Arzneistoffe herstellen, die nachfolgend mit Pellets (I) oder überzogenen Wirkstoffen (I) zu einer multipartikulären Arzneiform kombiniert werden können.

Es wurde überraschenderweise aber auch gefunden, dass sich diese sphärischen Granulate bzw. Partikel (II) nach Zumischung von nur wenig Außenphase, d.h. vor der Tablettierung nicht granuliert vorliegender Hilfsstoffe, wie sie vorstehend beschrieben wurden, zu Tabletten verpressen lassen. Dies ist eine spezielle Ausführungsvariante dieser Erfindung.

Gemäß einem weiteren Aspekt der vorliegenden Erfindung wird demgemäß eine Tablette bereitgestellt, welche enthält:

eine Innenphase, umfassend sphärische Partikel (II),
und eine Außenphase mit einem oder mehreren vor der Tablettierung nicht granuliert vorliegenden Hilfsstoffen, ausgewählt aus Sprengmitteln, Schmiermitteln, Fliessregulierungsmitteln, Füllmitteln und Bindemitteln, wobei das Gewicht der Außenphase nicht mehr als 25 Prozent des Gesamtgewichts der Tablette, beispielsweise nicht mehr als 20%, 17,5%, 15%, 12,5%, 10%, 7,5% oder 5% ausmacht.

Das Gewicht der Wirkstoff enthaltenden sphärischen Partikel (II) kann in diesen Ausführungsformen beispielsweise zwischen 75 und 95%, z.B. mindestens 77,5%, mindestens 80%, mindestens 85% oder mindestens 90% des Gesamtgewichts der Tablette ausmachen.

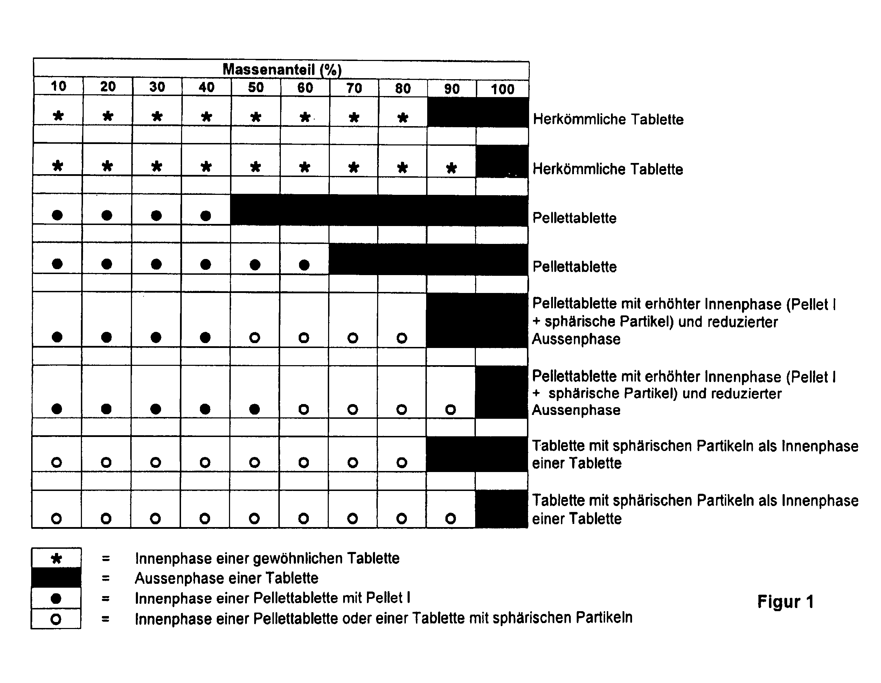

Eine graphische Darstellung von Phasenverhältnisse in Tabletten findet sich in Figur 1. Figur 1 stellt die prozentualen Massenanteile der Innen- und Außenphase von herkömmlichen, multipartikulären Tabletten sowie von Tabletten im Sinne dieser Erfindung dar.

Damit ergibt sich ein Anteil an Außenphase, der nicht mehr als 25 Prozent des Gesamtgewichts der Tablette, beispielsweise nicht mehr als 20%, 17,5%, 15%, 12,5%, 10%, 7,5% oder 5% ausmacht.

Es wurde auch überraschend gefunden, dass die sphärischen Partikel (II) beispielsweise mit dem zuvor beschriebenen speziellen Verfahren so hergestellt werden können, dass nach Tablettierung die Produkteigenschaften des ersten Pelletproduktes (I) oder die des überzogen Wirkstoffs (I) nur wenig bzw. praktisch nicht verändert werden und die daraus hergestellte Darreichungsform akzeptable Produkteigenschaften aufweist. Somit weisen die Unterschiede bei der Freisetzung von Pellets (I) nach Tablettierung mit sphärischen Partikeln (II) im Sinne der Erfindung von weniger als 20, auch weniger als 15, und insbesondere weniger als 10 % auf.

Gemäß einem weiteren Aspekt der Erfindung wird eine Tablette bereitgestellt, welche enthält:

eine Innenphase, umfassend

a) eine erste Sorte von Pellets (I), welche einen ersten Wirkstoff enthalten und welche eine Beschichtung aufweisen und/oder die Freisetzung des Wirkstoffs modifizieren, oder
b) eine erste Sorte überzogenen ersten Wirkstoffs (I),
im Gemisch mit
c) sphärischen Partikeln (II), die bevorzugt unter Verwendung von Feuchtgranulationsverfahren, insbesondere Wirbelschichtgranulationsverfahren, aber auch Rotorverfahren in der Wirbelschicht, und insbesondere eines Schaufelrotors hergestellt wurden,

wobei der Unterschied der Dissolutionsprofile der ersten Sorte von Pellets (I) oder der ersten Sorte überzogenen Wirkstoffs (I) nach Zumischen des sphärischen Aggregats (II) und nachfolgender Tablettierung weniger als 20 %, vorzugsweise weniger als 15 %, aber auch weniger als 10 % beträgt.

Mit anderen Worten weicht bei diesem Aspekt der Erfindung das Profil der Dissolution des ersten Wirkstoffs aus der ersten Sorte von Pellets(I) bzw. der ersten Sorte überzogenen Wirkstoffs (I), d.h. vor Zumischen der sphärischen Partikel (II) und vor nachfolgender Tablettierung, um weniger als 20 %, vorzugsweise weniger als 15 %, und am stärksten bevorzugt weniger als 10 % von dem Profil der Dissolution des ersten Wirkstoffs aus der erfindungsgemäßen Tablette ab.

Auch in dieser Ausführungsform können die sphärischen Partikel (II) einen Wirkstoff oder mehrere Wirkstoffe enthalten, welche(r) vorzugsweise von dem ersten Wirkstoff der Pellets (I) bzw. des überzogenen Wirkstoffs (I) verschieden ist. Eine vorteilhafte Ausführungsform betrifft mithin eine Tablette, die enthält:

eine erste Sorte von Pellets (I), welche einen ersten Wirkstoff enthalten und welche eine Beschichtung aufweisen und/oder den Wirkstoff modifiziert freisetzen, oder eine erste Sorte überzogenen ersten Wirkstoffs (I),
im Gemisch mit sphärischen Partikeln (II), welche unter Verwendung eines Schaufelrotors hergestellt wurden und einen zweiten Wirkstoff enthalten,
wobei der Unterschied der Dissolutionsprofile der ersten Sorte von Pellets (I) nach Zumischen des sphärischen Aggregats (II) und nachfolgender Tablettierung weniger als 20%, vorzugsweise weniger als 15%, aber auch weniger als 10% beträgt, z.B. weniger als 9%, 8%, 7%, 6%, 5%.

In den Ausführungsformen der vorliegenden Erfindung, in welchen die sphärischen Partikel (II) einen oder mehrere Wirkstoffe enthalten, beträgt der Wirkstoffanteil am Gesamtgewicht der sphärischen Partikel bevorzugt mindestens 70%, z.B. mindestens 75%, besonders bevorzugt 80%, 81%, 82%, 83%, 84%, 85%, 86%, 87%, 88%, 89% und am stärksten bevorzugt mindestens 90%, z.B. mindestens 91%, 92%, 93%, 94%, 95%, 96%, 97% oder 98%.

Der Gehalt der ersten Sorte von Pellets (I) an erstem Wirkstoff bzw. kumulative Gehalt der ersten Sorte von Pellets (I) an erstem Wirkstoff und weiterem Wirkstoff bzw. weiteren Wirkstoffen liegt im unbeschichteten Zustand vorzugsweise im Bereich von 50-100%. In bespielhaften Ausführungsformen liegt der Wirkstoffgehalt bei mindestens 60% oder 75%, oder mindestens 80%, 81%, 82%, 83%, 84%, 85%, 86%, 87%, 88%, 89% oder mindestens 90%, z.B. mindestens 91%, 92%, 93%, 94%, 95%, 96%, 97%, 98%, 99% bis zu 100 %. Beispielsweise bei Ibuprofen lässt sich ein 100%iger Wirkstoffgehalt realisieren.

Damit werden Herstellungs- bzw. Granulationsverfahren und entsprechende Tabletten auch für höher dosierte Wirkstoffe in Kombination mit Pelletprodukten bzw. überzogenen Wirkstoffen (I) mit höherem Wirkstoffanteil bereitgestellt, um gleichzeitig gute Verarbeitungs-, Zerfalls- und Auflösungseigenschaften aller arzneilich wirksamen Bestandteile einer Darreichungsform zu ermöglichen, die bei der Herstellung die Eigenschaften der inkorporierten Pellets (I), insbesondere die der Dissolution, im Wesentlichen unverändert lassen.

Mit Hilfe der vorliegenden Erfindung können insbesondere vorteilhafte Kombinationspräparate zweier oder mehrerer Wirkstoffe in Form von Tabletten bereitgestellt werden, in welchen zumindest ein Wirkstoff modifiziert freigesetzt wird, z.B. zwei Wirkstoffe modifiziert freigesetzt werden. Besonders vorteilhaft ist die vorliegende Erfindung ferner bei höherer Dosierung des Wirkstoffs bzw. der Wirkstoffe.

Bei der Herstellung dieser sphärischen Partikel (II), die den Pelletprodukten (I) zugemischt werden, können Teile der Außenphase einer multipartikulären Tablette, insbesondere der Anteil von mikrokristalliner Cellulose, durch sphärische Partikel (II) ersetzt werden, so dass sich das Phasenverhältnis von Innenphase zu Außenphase dieser multipartikulären Tablette dem einer klassischen Tablette angleicht. Durch die Einsparung an Außenphase kann selbst bei hohem Wirkstoffgehalt und bei Kombinationspräparaten eine Tablette mit akzeptablen Dimensionen und akzeptabler Schluckbarkeit erreicht werden.

Die mit dem oben beschriebenen Verfahren und der oben beschriebenen Zusammensetzung hergestellten sphärischen Partikel (II) weisen vorzugsweise einen Durchmesser zwischen 5 $\mu m$ und 1500 $\mu m$, insbesondere zwischen 50 $\mu m$ und 500 $\mu m$, beispielsweise kleiner als 400 $\mu m$, kleiner als 300 $\mu m$, größer als 100 $\mu m$ und größer als 150 $\mu m$ auf.

Die sphärischen Aggregate (II) sind vorzugsweise kugelig und weisen eine Sphärizität von 0,8 - 1,0, beispielsweise von 0,85 bis 1,0, von 0,9 bis 1,0 und insbesondere von 0,95 bis 1,0 auf. Die Sphärizität berechnet sich dabei nach der folgenden Formel:

$$SPHT = 4\pi A/U^2$$

mit A = Fläche und U = Umfang.

Die Sphärizität kann mit Geräten zur Partikelgrössen- und Partikelformanalyse mit dynamischer Bildanalyse durchgeführt werden. Ein hierfür geeignetes Gerät ist beispielsweise der CAMSIZER von Retsch.

Weiterhin ist es bevorzugt, dass das Verhältnis von Breite zu Länge der sphärischen Aggregate (II) im Bereich von 0,8 bis 1,0, insbesondere von 0,9 bis 1,0 liegt. Das Verhältnis von Breite zu Länge berechnet sich dabei nach folgender Formel:

$$b/l = min\ (x_C)/Max\ (x_{Fe})$$

mit $x_{Fe}$ = Feret-Durchmesser und $x_C$= maximale breite des Partikels.

Auch das Breite-Länge-Verhältnis lässt sich z.B. mit dem CAMSIZER von Resch bestimmen.

Die Schüttdichte, bestimmt nach den in gängigen Arzneibüchem dokumentierten Methoden, dieser mittels Schaufelrotor hergestellten sphärischen Aggregate ist $\leq$ 0,8 g/ml, z.B. $\leq$ 0,7 g/ml, vorzugsweise jedoch $\leq$ als 0,6 g/ml und insbesondere $\leq$ 0,5 g/ml.

Die absolute Porosität, das heißt der Prozentsatz des gesamten Hohlraumvolumens zum scheinbaren Volumen, der sphärischen Partikel (II) liegt vorzugweise liegt vorzugsweise in einem Bereich von 0,5 und 30 %, z.B. von 1 bis 20 %, von 1 bis 10 % oder von 2 bis 10 %.

Die vorstehend genannten bevorzugten Bereiche für die Eigenschaften der sphärischen Partikel (II) gelten alleine oder in Kombination ebenso als bevorzugt für die in den Tabletten verwendeten Pellets (I).

Nach dem Zumischen der sphärischen Partikel (II) zu einem Pelletprodukt (I) und nachfolgender Tablettierung ändert sich z.B. das Freisetzungsverhalten der Pellets (I), insbesondere der modifiziert freisetzenden Pellets (I), gegenüber den nicht tablettierten Pellets nur unwesentlich oder gar nicht, und die Tablettiermischung lässt sich zu Tabletten mit einer akzeptablen Gewichts- und Gehaltseinheitlichkeit sowie ausreichender mechanischer Festigkeit verarbeiten.

Beispiele für Wirkstoffe bzw. Wirkstoffklassen, welche vorteilhaft im Rahmen der vorliegenden Erfindung eingesetzt werden können, sind:

Arzneimittel zur Behandlung von Schmerzen und zur Schmerztherapie mit peripher wirkenden Analgetika, zentral wirkenden Analgetika und adjuvante Nicht-Analgetika. Es handelt sich hier um folgende Analgetika und adjuvante Stoffe, allein oder in Kombination:

Acetylsalicylsäure, Ibuprofen, Diclofenac, Indomethacin, Naproxen, Piroxicam, Paracetamol, Metamizol, Celecoxib, Parecoxib, Tramadol, Pethidin, Codein, Dihydrocodein, Piritramid, Tilidin, Morphin, Hydromorphon, Oxycodon, Levomethadon, Fentanyl, Sufentanil, Buprenorphin, Pentazocin, Naloxon, Flupirtin, Carbamazepin, Metoprolol, Metoclopramid, Amitryptilin, Doxepin, Clomipramin, Mianserin, Maprotilin, Triptane wie z.B. Naratriptan, Rizatriptan, Sumatriptan, Zolmitriptan, Kalziumantagonisten wie: Flunarizin, Topiramat, Valproinsäure, Phenytoin, Baclofen, sonstige Mittel wie: Botulinum Toxin, Ergotamine, Lisurid, Methysergid, Pizotifen, Oxcarbazepin, Gabapentin und Lamotrigin, Dexamethaon, Methylprednisolon, Prednisolon, Triamcinolon, Diazepam, Tetrazepam, Tizanidin, Butylscopolamin, Kombination von Tilidion und Naloxon.

Arzneistoffe/Arzneimittel zur Behandlung des Nervensystems, allein oder in Kombination, z.B. Anfallsleiden (Clonazepam, Diazepam, Lorazepam, Midazolam, Clobazam, Phenytoin, Clomethiazol, Valproinsäure, Phenobarbital, Gabapentin, Lamotrigin, Oxcarbazepin, Pregabalin, Topiramat, Ethosuximid, Levetrazetam, Mesuximid, Primidon, Nitrazepam, Vigabitrin), Parkinson-Syndrom (Levodopa, mit Benserazid/Carbidopa, Bromocriptin, Cabergolin, Dihydroergocriptin, Lisurid, Pergolidmesilat, Pramipexol, Ropinirol, Apomorphin, Biperiden, Metixenhydrochlorid, Trihexphenidyl, Entacapon, Amantadin, Bupidin, Selegilin, Apomorphin), Schlaganfall (Acetylsalicylsäure, Clopidogrel, Dipyridamol, Ticlopidin, Heparin, Phenprocoumon, Warfarin, Protamin, Phytomenadion, Nimodipin, Paracetamol, Tramadol, Buprenorphin), Hirndruck (Furosemid, Mannitol), Tremor (Propranolol, Clozapin, Alprazolam, Primidon). Arzneistoffe/Arzneimittel, allein oder in Kombination, zur Behandlung von physchiatrischen Erkrankungen wie Angststörungen (Alprazolam, Diazepam, Fluoxetin, Paroxetin, Chlorprothixen, Levomepromazin, Thioridazin, Flupentixol, Fluspirilen, etc.), Depressionen (Imipramin, Amitripytlin, Desipramin, Maprotilin, Minaserin, Citalopram, Fluoxetin, Paroxetin, Trazodon, Moclobemid, Miratazepam etc.,), Psychosen und Schizophrenien (Sulpirid, Promazin, Melperon, Thioridazin, Chlorprothixen, Perazin, Pimozid, Fluphenazin, Olanzapin, Risperidon, etc.), Schlafstörungen (Triazolam, Brotizolam, Oxazepam, Flurazepam, Nitrazepam, Temazepam, Zolpidemtratrat, Zopiclon, Promethazin, Chlorprothixen, Pipamperon, Thioridazin, Chloralhydrat, etc.) Unruhezustände und Venivirrtheit (Alprazolam, Oxazepam, Doxepin, Clomipramin, Imipramin, Thioridazin, Perazin, etc.), Demenz vom Alzheimer-Typ (Donepezil, Rivastigmin, Tacrin, Memantin, Nimodipin, Seleginin etc.).

Arzneistoffe/Arzneimittel zur Behandlung von Herz-Kreislauf Krankheiten, allein oder in Kombination, wie Koronare Herzkrankheit/Angina Pectoris (Acetylsalicylsäure, Clopidrogel, Ticlopidin, Isosorbiddinitrat, Isosorbidmononitrat, Nitroglycerin, Molsidomin, Trapidil, Metoprolol, Bisoprolol, Atenolol, Acebutolol, Carvedilol, Nitrendipin, Nifedipin, Diltiazem, Verapamil, Benazepril, Lisinopril, Ramipril, Fosinopril, Enalapril, etc.), Herzinfarkt und Herzinsuffizienz (Isosorbidmono- und dinitrat, Clopidogrel, Ticlopidin, Captoprol, Ramipril, Lisinopril, Candesartan, Eproartan, Irbesatan, Losartan, Chlortalidon, Xipamid, Hydrochlorothiazid, Furosemid, Piretanid, Triameteren, Digitalisglycoside, Carvedilol, Metoprolol, Prazosin, etc.), Herzrhythmusstörungen (Ajmalin, Chinidin, Disopyramid, Flecainid, Propafenon, Propranolol, Carvedilol, Amiodaron, Verapamil, Diltiazem, etc.), Hypertonie (Metoprolol, Atenolol, Urapidil, Clonidin, Dihydralazin, Chlortalidon, Hydrochloroithiazid, Furosemid, Felodipin, Israpidin, Lacidipin, Diltiazem, Captopril, Enalapril, Fosinopril, Lisinopril, Ramnipril, Verapamil, Candesartan, Eprosartan, Irbesatan, Losartan, Doxazosin, Bunazosin, Prazosin, Terazosin, Moxonidin, Dihydralazin, Minoxidil).

Arzneistoffe/Arzneimittel, allein oder in Kombination, zur Behandlung der Atemwege und der Lunge (Theophyllin, Methylprednisololn, Flucortoln, Dexamethason, Montekulast, Roxithromycin, Erythromycin, Azithromycin, Ciprofloxacin, Clarithromycin, Levofloxacin, Ofloxacin, Doxycyclin, Ampicillin und Sulbactam, Amoxicillin, Cefuroxim, Clindamycin, Cefotiam, Cefuroxim, Cefotiam, Ceftazidim, Ceftriaxon, Piperacillin, Moxifloxacin, etc.).

Arzneistoffe/Arzneimittel, allein oder in Kombination, zur Behandlung des Magen-Darm-Traktes und der Bauspeicheldrüse (Fluconazol, Mesalazin, Sulfasalazin, Budenosid, Azathioprin, Prednison, Metronidazol, Infliximab, Loperamid, Cotrimoxazol, Ciprofloxacin, Metronidazol, Vancomycin, Esomeprazol, Lansoparzol, Pantoprazol, Rabeprazol, Cimetidin, Famotidin, Ranitidin, Nizatidin, Sucralfat, Misoprostol, Metoclopramid, Pirenzepin, Plantago-Samen, Bisacodyl, Domperidon, Sulpirid, Alizaprid, Dimenhydrinat, Cinnarizin, Flunarizin, Levomeprazin, Ondansetron, Betahsitidin, Aprepitant, etc.).

Arzneistoffe/Arzneimittel, allein oder in Kombination, zur Infektabwehr mit Antibiotika oder antiviral wirksamen Substanzen (Acyclovir, Amantadin, Azithromycin, Bacampicillin, Cefaclor, Cefazolin, Cefixim, Cefprozil, Ceftriaxon, Chloroquin, Ciprofloxacin, Clotrimazol, Dicloxacillin, Doxycycllin, Econazol, Erythromycin, Ethambutol, Fosfomycin, Flucloxacillin, Fluconazol, Fusidinsäure, Gramicidin, Idoxuridin, Indinavir, Interferon, Itraconazol, Isoniazid, Josamycin, Ketoconazol, Lamivudin, Lomefloxacin, Mafenid, Mebendazol, Mesalazin, Mezlozillin, Mupirocin, Miconazol, Naftifin, Nalidixinsäure, Norfloxacin, Ofloxacin, Oxacillin, Oxytetracyclin, Piperacillin, Praziquantel, Primaquim, Proguanil, Ribavirin, Rifabutin, Rimantadin, Roxothromycin, Saquinavir, Spectinomycin, Spriramycin, Stavudin, Sulbactam, Teiucoplanin, Terbinafin, Tetracyclin, Tetroxoprim, Ticarcillin, Tinidazol, Tromantadin, Tolnaftat, Vancomycin, Zidovudin, Zalcitabin, etc.).

Arzneistoffe/Arzneimittel zur Behandlung der erktilen Dysfunktion, allein oder in Kombination (Sildenafil, Tadalafil, Vardenafil, Theobromin, Koffein, Theophyllin, etc.).

Kombinationspräparate sind insbesondere Kombinationen von Wirkstoffen zur Behandlung von cardiovaskulären Erkrankungen, wie z.B. Bluthochdruck, zur Behandlung des Zentralen Nerven Systems (ZNS), wie z.B. der Parkinsonschen Krankheit oder Depressionen, zur Infektabwehr mit Antibiotika oder antiviral wirksamen Substanzen, zur oralen Empfängnisverhütung, zur Behandlung von Magenerkrankungen und in der Schmerztherapie.

Die Tablette kann beispielsweise eine oral dispersible Tablette (oral dispersible tablet) sein. Alternativ kann die Tablette auch klassisch formuliert erst in tieferen Abschnitten des Magen-Darm-Traktes in ihre Untereinheiten zerfallen.

Die Erfindung wird nachfolgend unter Bezugnahme auf mehrere Ausführungsbeispiele und die folgenden Figuren beschreiben. Dabei zeigt

Figur 1 die prozentualen Massenanteile der Innen- und Außenphase von herkömmlichen, multipartikulären Tabletten sowie von Tabletten im Sinne dieser Erfindung;

Figur 2 das Dissolutionsprofil einer Tablette gemäß eines ersten Ausführungsbeispiels der Erfindung im Vergleich zum Dissolutionsprofil der verwendeten Pellets; und

Figur 3 das Dissolutionsprofil einer Tablette gemäß eines zweiten Ausführungsbeispiels der Erfindung im Vergleich zum Dissolutionsprofil der verwendeten Pellets;

Figur 4 das Dissolutionsprofil einer Tablette gemäß eines dritten Ausführungsbeispiels der Erfindung im Vergleich zum Dissolutionsprofil der verwendeten Pellets.

Beispiel 1:

| Modifiziert freisetzende Ibuprofen Tabletten | | |
|---|---|---|
| Komponente | Einzeldosis | |
| Pellet | Masse (mg) | Anteil (%) |
| Ibuprofen Pellets | 200,0 | 28,57 |
| Retardierender Filmüberzug | | 4,29 |
| Ethocel Standard 10 Premium | 30,0 | |
| HPC-L | 30,0 | 4,29 |
| Sphärische Partikel | | |
| Pearlitol 25 C | 391,3 | 55,90 |
| Kollidon CL-M | 43,5 | 6,21 |
| Außenphase | | 0,75 |
| Natriumstearylfumarat | 5,3 | |
| Total | 700,00 | 100,00 |

300 -1000 g Ibuprofen Pellets der Teilchengrösse 100-200 $\mu$m werden in einer Wirbelschichtapparatur mit Wurstereinsatz (GPCG 1, Fa. Glatt, mit 6" Wurstereinsatz) mit einer Lösung enthaltend 2,5 % Ethocel Standard 10 Premium von Colorcon und 2,5 % HPC-L von Nisso in Ethanol bei einer Produkttemperatur von 30 °C und einer Sprührate von etwa 5-10 g/min beschichtet.

Zur Herstellung der sphärischen Partikel werden Kollidon CL-M von BASF und Pearlitol 25 C von Roquette im Massenverhältnis 10:90 im Schnellmischer, VG 10, Fa. Glatt, gemischt und homogen mit einer 15 % Lösung von Pearlitol 160 C in Wasser vorbefeuchtet. Die Restfeuchte dieses Produktes beträgt etwa 10 % (Mettler Halogen Trockner, etwa 5 g, 105 C, 1 mg/30 sec). Nachfolgend wird das vorbefeuchtete Material im Schaufelrotor zu sphärischen Partikeln unter Zusatz von demineralisiertem Wasser sphäronisiert, bis die mittlere Korngrösse etwa 200 $\mu$m beträgt. Der Schaufelrotor, CPS 3, Fa. Glatt, ist dazu mit einer 30 ° Rotorplatte ausgestattet. Im Prozessraum befinden sich vier flache Schaufeln, um die Gutbewegung entsprechend auszurichten. Die Zulufttemperatur während der Sphäronisation beträgt 35°C. Die Luftmenge etwa 75 m$^3$/h. Die Rotorgeschwindigkeit beträgt 350 UpM. Die Restfeuchte dieses Materials beträgt 20 %, bestimmt nach dem oben angegebenen Verfahren. Die so erhaltenen sphärischen Partikel werden nachfolgend in der Wirbelschicht bei einer Zulufttemperatur von 60 °C getrocknet bis die Restfeuchtigkeit des getrockneten Produktes einen Wert von etwa < 0,2 % erreicht. Die Schüttdichte dieser spärischen Partikel beträgt 0,6 g/ml.

Die überzogenen Ibuprofen Pellets werden im oben angegeben Verhältnis mit den sphärischen Partikeln bestehend aus Pearlitol 25 C und Kollidon CL-M und dem Natriumstearylfumarat (Pruv, JRS) im Turbula T2C (Bachofen) gemischt.

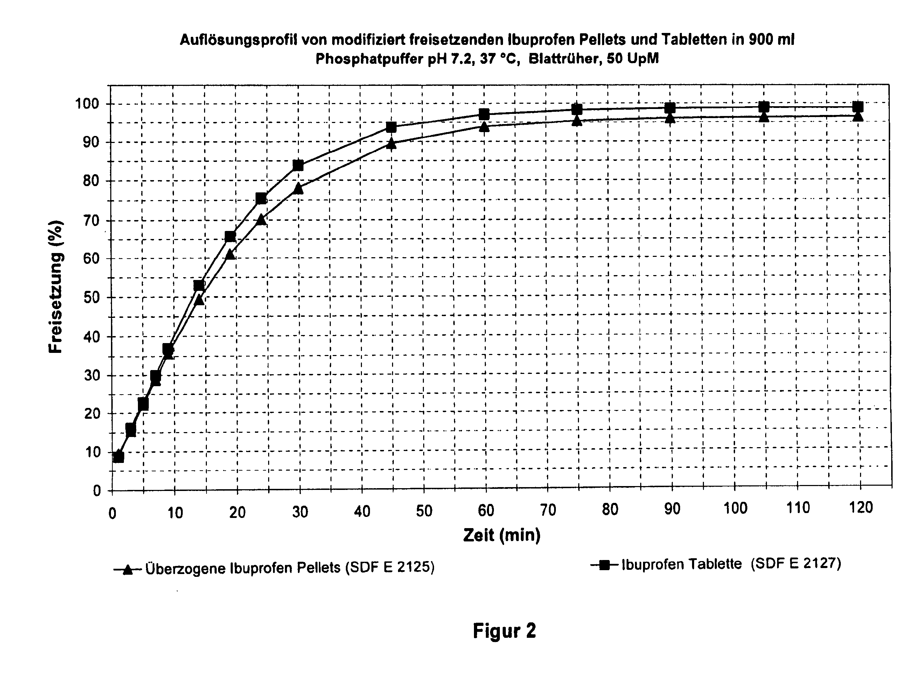

Nachfolgend werden Tabletten mit einem Durchmesser von 16 mm, mit einer Tablettenpresse, z.B. Korsch EK 0, hergestellt. Die Härte der Tabletten beträgt 30 N. Das Freisetzungsverhalten der überzogenen Pellets und der Tablette ist in Figur 2 wiedergegeben.

Beispiel 2:

| Geschmacksmaskierte Acetylsalicylsäure Tabletten | | |
|---|---|---|
| Komponente | Einzeldosis | |
| Wirkstoff | Massse (mg) | Anteil (%) |
| Acetylsalicyclsäure | 250,0 | 41,67 |
| Geschmacksmaskierender Uberzug | | |
| Ethocel Standard 10 Premium | 30,0 | 5,00 |
| HPC-L | 7,5 | 1,25 |

(fortgesetzt)

| Geschmacksmaskierte Acetylsalicylsäure Tabletten | | |
|---|---|---|
| Komponente | Einzeldosis | |
| Wirkstoff | Massse (mg) | Anteil (%) |
| Sphärische Partikel | | |
| Pearlitol 25 C | 262,4 | 43,73 |
| Kollidon CL-M | 29,2 | 4,86 |
| Außenphase | | |
| Aerosil 200 | 3,0 | 0,50 |
| Lubritab | 18,0 | 3,00 |
| Total | 600,00 | 100,00 |

750 g Acetylsalicylsäure wird in der Wirbelschicht mit einem geschmacksmaskierenden Überzug aus 80 % Ethocel Standard 10 Premium (Fa. Colocon) und 20 % HPC-L (Fa. Nisso) in der Wirbelschicht im Topsprayverfahren bei einer Produkttemperatur von 30°C besprüht. Die Komponenten (Ethocel und HPC) werden in Ethanol gelöst. Der Feststoffanteil dieser Sprühlösung beträgt 5 %.

Zur Herstellung der sphärischen Partikel werden Kollidon CL-M von BASF und Pearlitol 25 C von Roquette im Massenverhältnis 10:90 im Schnellmischer VG 10, Fa. Glatt, gemischt und homogen mit einer 15 %igen Lösung von Pearlitol 160 C in Wasser vorbefeuchtet. Die Restfeuchte dieses Produktes beträgt etwa 10 % (Mettler Halogen Trockner, etwa 5 g, 105 °C, 1 mg/30 sec). Nachfolgend wird das vorbefeuchtete Material im Schaufelrotor zu sphärischen Partikeln unter Zusatz von demineralisiertem Wasser sphäronisiert, bis die mittlere Korngrösse etwa 200 μm beträgt. Der Schaufelrotor, CPS 3, Fa. Glatt, ist dazu mit einer 30 ° Rotorplatte ausgestattet. Im Prozessraum befinden sich vier flache Schaufeln, um die Gutbewegung entsprechend auszurichten. Die Zulufttemperatur während der Sphäronisation beträgt 35 °C. Die Luftmenge etwa 75 m$^3$/h. Die Rotorgeschwindigkeit beträgt 350 UpM. Die Restfeuchte dieses Materials beträgt etwa 20 %, bestimmt nach dem oben angegebenen Verfahren. Die Pellets werden nachfolgend in der Wirbelschicht bei einer Zulufttemperatur von 60 °C getrocknet bis die Restfeuchtigkeit des getrockneten Produktes etwa < 0,2 % erreicht. Die Schüttdichte dieser sphärischen Partikel beträgt 0,6 g/ml.

Die überzogene Acetylsalicylsäure wird im oben angegeben Verhältnis mit den sphärischen Partikeln bestehend aus Pearlitol 25 C und Kollidon CL-M und Lubritab (JRS) und Aerosil 200 (Evonik) im Turbula T2C (Bachofen) gemischt.

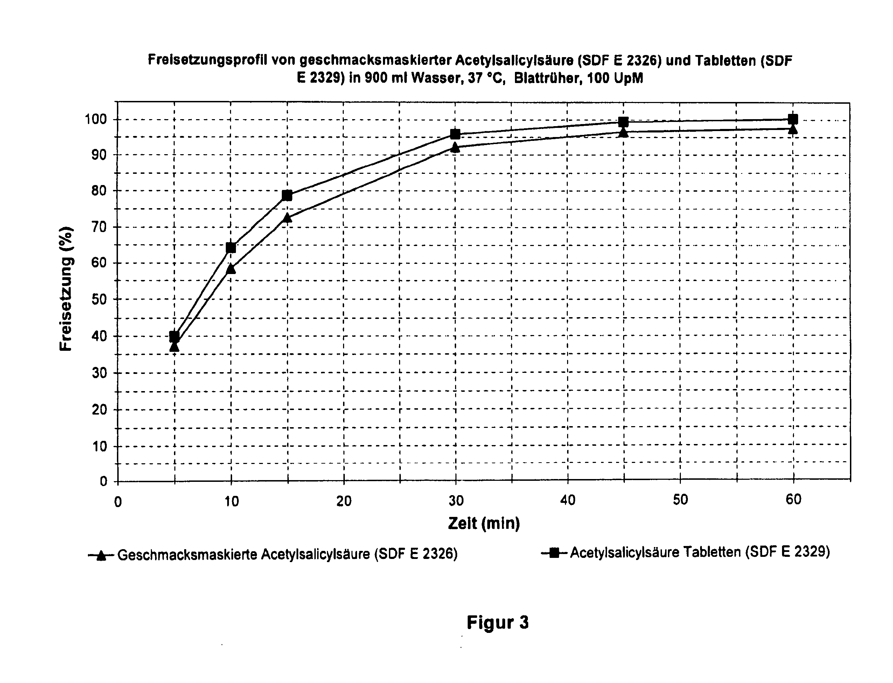

Nachfolgend werden Tabletten mit einem Durchmesser von 13 mm mit einer Tablettenpresse, z.B. Korsch EK 0, hergestellt. Die Härte der Tabletten beträgt 30 N. Das Freisetzungsverhalten der überzogenen Acetlysalicylsäure und der daraus hergestellten Tablette ist in Figur 3 wiedergegeben.

Beispiel 3:

| Acetylsalicylsäure/Koffein Tablette | | |
|---|---|---|
| Komponente | Einzeldosis | |
| Koffein Pellets | Masse (mg) | Anteil (%) |
| Koffein | 50,0 | 8,82 |
| Pearlitol 25 C | 14,3 | 2,52 |
| Kollidon K 30 | 7,1 | 1,25 |
| Geschmacksmaskierender Überzug | | |
| Ethocel Standard 10 Premium | 17,1 | 3,02 |
| HPC-L | 4,3 | 0,76 |
| Sphärische Acetylsalicyclsäuepartikel | | |

(fortgesetzt)

| Acetylsalicylsäure/Koffein Tablette | | |
|---|---|---|
| **Komponente** | **Einzeldosis** | |
| **Koffein Pellets** | **Masse (mg)** | **Anteil (%)** |
| Acetylsalicylsäure | 250,0 | 44,09 |
| Pearlitol 25 C | 73,3 | 12,93 |
| Kollidon K 30 | 37,5 | 6,61 |
| **Außenphase** | | |
| Kollidon CL | 56,7 | 10,00 |
| Pearlitol 160 C | 49,6 | 8,75 |
| Aerosil 200 | 2,8 | 0,50 |
| Natriumstearylfumarat | 4,3 | 0,75 |
| **Total** | 567,0 | 100,00 |

Im Schnellmischer werden Koffein, Pearlitol 25 C (Roquette), Kollidon K 30 (BASF) gemischt und mit einer wässrigen Lösung von Kollidon K 30 (10 %) und Pearlitol 25 C (10 %), vorbefeuchtet. Die Restfeuchte des Materials beträgt 6 % (Mettler Halogentrockner, 105 °C, 5 g, 1 mg/30sec). Die so vorbefeuchtete Masse wird in einem Schaufelrotor unter gleichzeitigem Besprühen mit Wasser zu Pellets verrundet. Der Schaufelrotor, CPS 3, Fa. Glatt, ist dazu mit einer 45 ° Rotorplatte ausgestattet. Im Prozessraum befinden sich vier flache Schaufeln, um die Gutbewegung entsprechend auszurichten. Die Zulufttemperatur während der Sphäronisation beträgt 35 °C. Die Luftmenge etwa 30 m³/h. Die Rotorgeschwindigkeit 800 +/- 100 UpM. Die so entstandenen Koffein-Pellets werden bei 60 °C Zulufttemperatur in der Wirbelschicht (Glatt, GPCG 1) getrocknet. Die Restfeuchte des Materials beträgt 0.4 %. Die mittlere Korngrösse. bestimmt mittels Siebanalyse, beträgt etwa 200 μm.

Zur Geschmacksmaskierung werden 300 - 1000 g Koffein-Pellets mit einem geschmacksmaskierenden Überzug aus 80 % Ethocel Standard 10 Premium (Fa. Colorcon) und 20 % HPC-L (Fa. Nisso) in der Wirbelschicht mit Wurstereinsatz (GPCG1, Glatt, 6 " Wurster) bei einer Produkttemperatur von 30 °C überzogen. Die Komponenten (Ethocel und HPC) werden in Ethanol gelöst. Der Feststoffanteil der Lösung beträgt 5 %.

Im Schnellmischer werden Acetylsalicylsäure, Pearlitol 25 C, Kollidon K 30 gemischt und mit einer wässrigen Lösung von Kollidon K 30 (10 %) und Pearlitol 25 C (10 %), vorbefeuchtet. Die so vorbefeuchtete Masse wird in einem Schaufelrotor unter gleichzeitigem Besprühen mit Wasser zu sphärischen Partikeln verrundet. Der Schaufelrotor, CPS 3, Fa. Glatt, ist dazu mit einer 30 ° Rotorplatte ausgestattet. Im Prozessraum befinden sich vier flache Schaufeln, um die Gutbewegung entsprechend auszurichten. Die Zulufttemperatur während der Sphäronisation beträgt 35 °C. Die Luftmenge etwa 50 m³/h. Die Rotorgeschwindigkeit beträgt 500 UpM. Die so entstandenen Acetylsalicylsäure-Pellets werden bei 60 °C Zulufttemperatur in der Wirbelschicht (Glatt, GPCG 1) getrocknet. Die Restfeuchte des Materials beträgt < 0,2 %. Die mittlere Korngrösse, bestimmt mittels Siebanalyse, beträgt etwa 125 μm. Die Schüttdichte beträgt etwa 0,5 g/ml.

Die überzogenen Koffein-Pellets werden im oben angegebenen Verhältnis mit den sphärischen Acetylsalicylsäure-Partikeln, die neben dem Wirkstoff aus Pearlitol 25 C (Roquette) und Kollidon K 30 (BASF) bestehen, sowie den Komponeneten der Außenphase (Kollidon CL (BASF), Pearlitol 160 C (Roquette), Aerosil 200 (Evonik) und Natriumstearylfumarat (Pruv, JRS) im Turbula T2C (Bachofen)) gemischt.

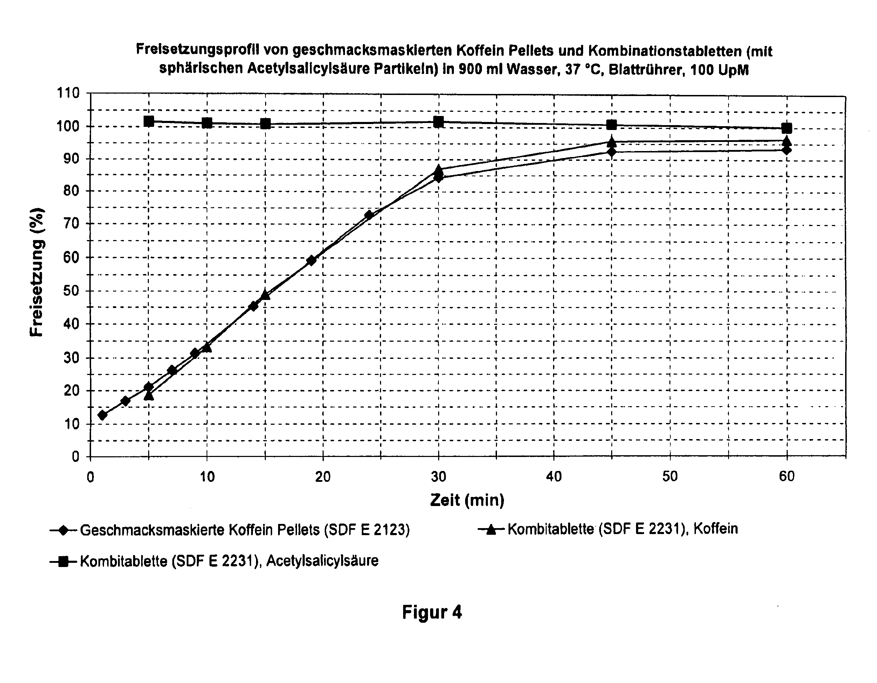

Nachfolgend werden Tabletten mit einer Tablettenpresse, z.B. Korsch EK 0, Tabletten mit einem Durchmesser von 13 mm hergestellt. Die Härte der Tabletten beträgt 30 N. Das Freisetzungsverhalten der überzogenen Koffeinpellets und der daraus hergestellten Tabletten ist in Figur 4 wiedergegeben.

**Patentansprüche**

1.  Tablette, enthaltend
    eine Innenphase, umfassend

    a) eine erste Sorte von Pellets (I), welche einen ersten Wirkstoff enthalten und welche eine Beschichtung aufweisen und/oder den Wirkstoff modifiziert freisetzen, oder
b) eine erste Sorte überzogenen ersten Wirkstoffs (I),
im Gemisch mit
c) sphärischen Partikeln (II),

und eine Außenphase mit einem oder mehreren vor der Tablettierung nicht granuliert vorliegenden Hilfsstoffen, ausgewählt aus Sprengmitteln, Schmiermitteln, Fließregulierungsmitteln, Füllmitteln und Bindemitteln, wobei das Gewicht der Außenphase nicht mehr als 25 Prozent des Gesamtgewichts der Tablette ausmacht und wobei die sphärischen Partikel (II) eine Dichte von kleiner als 0,8 g/ml aufweisen.

2. Tablette gemäß Anspruch 1,
wobei die erste Sorte von Pellets (I) mit einem Film überzogen ist, welcher die Freisetzung des ersten Wirkstoffs aus den Pellets (I) modifiziert.

3. Tablette gemäß Anspruch 1,
wobei der Überzug des ersten Wirkstoffs ein Überzug ist, welcher die Auflösung des Wirkstoffs modifiziert.

4. Tablette gemäß einem der vorangehenden Ansprüche, wobei die sphärischen Partikel (II) einen zweiten Wirkstoff enthalten.

5. Tablette, enthaltend:

eine erste Sorte von Pellets (I), welche einen ersten Wirkstoff enthalten und welche eine Beschichtung aufweisen und/oder den Wirkstoff modifiziert freisetzen, oder eine erste Sorte überzogenen ersten Wirkstoffs (I), im Gemisch mit sphärischen Partikeln (II), wobei der Unterschied der Dissolutionsprofile der ersten Sorte von Pellets (I) nach Zumischen der sphärischen Partikel (II) und nachfolgender Tablettierung weniger als 20 % beträgt und wobei die sphärischen Partikel (II) eine Dichte von kleiner als 0,8 g/ml aufweisen.

6. Tablette, enthaltend:

eine Innenphase, umfassend sphärische Partikel (II), welche einen Wirkstoff enthalten, und eine Außenphase mit einem oder mehreren vor der Tablettierung nicht granuliert vorliegenden Hilfsstoffen, ausgewählt aus Sprengmitteln, Schmiermitteln, Fließregulierungsmitteln, Füllmitteln und Bindemitteln, wobei das Gewicht der Außenphase nicht mehr als 25 Prozent des Gesamtgewichts der Tablette, beispielsweise nicht mehr als 20%, 17,5%, 15%, 12,5%, 10%, 7,5% oder 5% ausmacht und wobei die sphärischen Partikel (II) eine Dichte von kleiner als 0,8 g/ml aufweisen.

7. Tablette gemäß Anspruch 6, wobei der Anteil des Wirkstoffs am Gewicht der sphärischen Partikel (II) mindestens 80% beträgt.

8. Tablette gemäß einem der vorangehenden Ansprüche, wobei die sphärischen Partikel (II) mittels eines Feuchtgranulationsverfahrens hergestellt sind.

9. Tablette gemäß Anspruch 8, wobei die sphärischen Partikel mittels Rotorgranulation hergestellt sind.

10. Tablette gemäß Anspruch 9, wobei die sphärischen Partikel unter Verwendung eines Schaufelrotors hergestellt sind.

**Claims**

1. Tablet, containing

a) an internal phase, comprising a first variety of pellets (I) containing a first active substance and comprising a coating and/or giving off the active substance in a modified manner, or
b) a first variety of a first coated active substance (I),
mixed with
c) spherical particles (II),
and an external phase containing one or more active substance/s whose condition prior to the tableting process is not a granulated one, chosen from blasting means, lubricants, flow controlling media, filling and binding agents, wherein the weight of the external phase does not exceed 25 per cent of the tablet's gross weight and the spherical particles (II) have a density of less than 0,8 g/ml.

2.  Tablet according to claim 1, wherein the first variety of pellets (I) is coated with a film modifying release of the first active substance from the pellets (I).

3.  Tablet according to claim 1, wherein the coat of the first active substance is a coat that modifies the dissolution of the active substance.

4.  Tablet according to one of preceding claims, wherein the spherical particles (II) contain a second active substance.

5.  Tablet, containing:

    a first variety of pellets (I) which contain a first active substance and which have a coating and/or give off the active substance in a modified manner, or a first variety of a coated first active substance (I), mixed with spherical particles (II), wherein the difference of dissolution profiles of the first variety of pellets (I) after adding of spherical particles (II) and subsequent tableting process is less than 20 %, and wherein the spherical particles (II) have a density of less than 0,8 g/ml.

6.  Tablet, containing:

    an internal phase comprising spherical particles (II) which contain an active substance, and an external phase comprising one or more excipient/s whose condition before tableting process is not a granulated one, chosen from blasting means, lubricants, flow controlling media, filling and binding agents, wherein the weight of the external phase does not exceed 25 per cent of the tablet's gross weight, for instance not exceeding 20%, 17,5%, 15%, 12,5%, 10%, 7,5% or 5% and wherein the spherical particles (II) have a density of less than 0,8 g/ml.

7.  Tablet according to claim 6 wherein the proportion of the active substance of the weight of the spherical particles (II) is at least 80 %.

8.  Tablet according to one of preceeding claims, wherein the spherical particles (II) are produced by means of a humid granulation method.

9.  Tablet according to claim 8 wherein the spherical particles are produced by means of a rotary granulation method.

10. Tablet according to claim 9 wherein the spherical particles are produced using a blade rotor.

**Revendications**

1.  Comprimé, contenant une phase intérieure, comprenant

    a) une première sorte de pellets (I) qui contiennent une première substance active et sont enduits et/ou dégagent la substance active de façon modifiée, ou
    b) une première sorte de substance active enduite (I), en mélange avec
    c) des particules sphériques (II),

    et une phase extérieure comprenant un ou plusieurs agent/s auxiliaire/s non pas présents sous forme granulée avant le procédé de compression, choisis dans les explosifs, lubrifiants, agents de contrôle fluidique, agents de charge et liants, dans lequel le poids de la phase externe n'excède pas les 25 pour cent du poids total du comprimé et les particules sphériques (II) ont une densité inférieure à 0,8 g/ml.

2.  Comprimé selon la revendication 1,

dans lequel la première sorte de pellets (I) est enduite d'un film modifiant la libération de la première substance active depuis les pellets (I).

3. Comprimé selon la revendication 1,
dans lequel l'enduit de la première matière active est un enduit modifiant la dissolution de la matière active.

4. Comprimé selon l'une des revendications précédentes,
dans lequel les particules sphériques (II) contiennent une deuxième substance active.

5. Comprimé, contenant:

une première sorte de pellets (I) qui contiennent une première substance active et présentent un enduit et/ou dégagent la substance active de façon modifiée, ou une première sorte d'une première substance active enduite, en mélange avec des particules sphériques (II),
dans lequel la différence des profils de dissolution de la première sorte de pellets (I) est, après ajout des particules sphériques (II) et la transformation en comprimés, est inférieure à 20 %, les particules sphériques (II) ayant une densité inférieure à 0,8 g/ml.

6. Comprimé, contenant:

une phase intérieure comportant des particules sphériques (II) qui contiennent une substance active, et une phase extérieure qui contient un ou plusieurs agent/s auxiliaire/s non pas présent/s sous forme granulée avant la transformation en comprimé, choisis dans les explosifs, lubrifiants, agents de contrôle fluidique, agents de charge et liants,
dans lequel le poids de la phase externe n'excède pas les 25 pour cent du poids total du comprimé, par exemple n'excédant pas 20 %, 17,5 %, 15 %, 12,5 %, 10 % ou 5 %, les particules sphériques (II) ayant une densité inférieure à 0,8 g/ml.

7. Comprimé selon la revendication 6 dans lequel la proportion de la substance active au poids des particules sphériques (II) s'élève à au moins 80 %.

8. Comprimé selon l'une des revendications précédentes dans lequel les particules sphériques (II) sont fabriquées moyennant un procédé de granulation humide.

9. Comprimé selon la revendication 8 dans lequel les particules sphériques sont fabriquées moyennant un procédé de granulation rotationnelle.

10. Comprimé selon la revendication 9 dans lequel les particules sphériques sont fabriquées grâce à la mise en oeuvre d'un rotor à aubes.

| Massenanteil (%) | | | | | | | | | | |
|---|---|---|---|---|---|---|---|---|---|---|
| 10 | 20 | 30 | 40 | 50 | 60 | 70 | 80 | 90 | 100 | |
| * | * | * | * | * | * | * | * | ■ | ■ | Herkömmliche Tablette |
| * | * | * | * | * | * | * | * | * | ■ | Herkömmliche Tablette |
| ● | ● | ● | ● | ■ | ■ | ■ | ■ | ■ | ■ | Pellettablette |
| ● | ● | ● | ● | ● | ● | ■ | ■ | ■ | ■ | Pellettablette |
| ● | ● | ● | ● | ○ | ○ | ○ | ○ | ■ | ■ | Pellettablette mit erhöhter Innenphase (Pellet I + sphärische Partikel) und reduzierter Aussenphase |
| ● | ● | ● | ● | ● | ○ | ○ | ○ | ○ | ■ | Pellettablette mit erhöhter Innenphase (Pellet I + sphärische Partikel) und reduzierter Aussenphase |
| ○ | ○ | ○ | ○ | ○ | ○ | ○ | ○ | ■ | ■ | Tablette mit sphärischen Partikeln als Innenphase einer Tablette |
| ○ | ○ | ○ | ○ | ○ | ○ | ○ | ○ | ○ | ■ | Tablette mit sphärischen Partikeln als Innenphase einer Tablette |

| | | |
|---|---|---|
| * | = | Innenphase einer gewöhnlichen Tablette |
| ■ | = | Aussenphase einer Tablette |
| ● | = | Innenphase einer Pellettablette mit Pellet I |
| ○ | = | Innenphase einer Pellettablette oder einer Tablette mit sphärischen Partikeln |

Figur 1

Auflösungsprofil von modifiziert freisetzenden Ibuprofen Pellets und Tabletten in 900 ml
Phosphatpuffer pH 7.2, 37 °C, Blattrüher, 50 UpM

—▲— Überzogene Ibuprofen Pellets (SDF E 2125)        —■— Ibuprofen Tablette  (SDF E 2127)

**Figur 2**

EP 2 346 493 B1

**Freisetzungsprofil von geschmacksmaskierter Acetylsalicylsäure (SDF E 2326) und Tabletten (SDF E 2329) in 900 ml Wasser, 37 °C, Blattrüher, 100 UpM**

—▲— Geschmacksmaskierte Acetylsalicylsäure (SDF E 2326)       —■— Acetylsalicylsäure Tabletten (SDF E 2329)

**Figur 3**

EP 2 346 493 B1

**Freisetzungsprofil von geschmacksmaskierten Koffein Pellets und Kombinationstabletten (mit sphärischen Acetylsalicylsäure Partikeln) in 900 ml Wasser, 37 °C, Blattrührer, 100 UpM**

—◆— Geschmacksmaskierte Koffein Pellets (SDF E 2123)      —▲— Kombitablette (SDF E 2231), Koffein

—■— Kombitablette (SDF E 2231), Acetylsalicylsäure

**Figur 4**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2004052607 A1


**In der Beschreibung aufgeführte Nicht-Patentliteratur**

- **R. VOIGT.** *Pharmazeutische Technologie,* 1995, vol. 8
- **K.H. BAUER ; K.-H. FRÖMMING ; C. FÜHRER.** *Pharmazeutische Technologies,* 1986
- **M. BRAUN.** *Dissertation,* 2003
- **K.G. WAGNER.** *Dissertation,* 1999